# EUROPÄISCHE PATENTANMELDUNG

(11) **EP 0 238 824 A1**
(43) Veröffentlichungstag der Anmeldung: **30.09.1987**
(21) Anmeldenummer: 87101794.3
(22) Anmeldetag: 10.02.1987
(51) Int. Cl.: C07D 233/56, C07D 249/18, A01N 43/50, A01N 43/647

(54) **Mikrobizide Mittel**

(30) Priorität: 22.02.1986 DE 3605714
(71) Anmelder: BAYER AG, 51368 Leverkusen (DE)
(72) Erfinder: Riebel, Hans-Jochem, Dr., D-5600 Wuppertal 1 (DE); Yanagi, Akihiko, Dr., Oume-shi Tokyo (JP); Hirashima, Akinori, Dr., Yame-City Fukuoka 834 (JP); Brandes, Wilhelm, Dr., D-5653 Leichlingen 1 (DE); Reinecke, Paul, Dr., D-5090 Leverkusen 3 (DE)

(57) **Zusammenfassung**

Die teilweise bekannten Phenylsulfonylazole der Formel 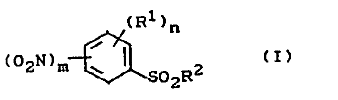 in welcher
R¹ für Alkyl steht,
m für die Zahlen 1 oder 2 steht,
n für die Zahlen 0 oder 1 steht und
R² für einen Rest der Formel 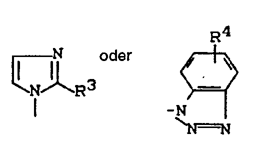 steht, worin
R³ für Alkyl steht und
R⁴ für Wasserstoff, Halogen, Nitro oder Alkyl steht, besitzen sehr gute mikrobizide Eigenschaften. Neue Phenylsulfonylazole der Formel 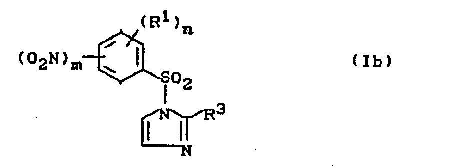
in welcher
R¹, R³, m und n die oben angegebenen Bedeutungen haben, sowie ein Verfahren zu deren Herstellung.

## Beschreibung

Die vorliegende Erfindung betrifft die Verwendung von teilweise bekannten Phenylsulfonylazolen als Mikrobizide.

Es ist bereits bekannt, daß bestimmte Phenylsulfonylimidazole als Bleichmittel eingesetzt werden können (vgl. US-PS 4 115 060). So läßt sich z.B. 1-(4-Methylphenylsulfonyl)-imidazol zum Reinigen und Aufhellen von Textilien verwenden.

Weiterhin ist bekannt, daß bestimmte N-Halogen-alkylthioimide fungizide Eigenschaften besitzen (vgl. EP-OS 0 044 394). So kann z.B. N-Trichlormethylthiotetrahydrophthalimid zur Bekämpfung von Pilzen eingesetzt werden. Die Wirkung dieses Stoffes ist jedoch bei niedrigen Aufwandmengen nicht immer voll befriedigend.

Außerdem sind bestimmte Phenylsulfonylbenztriazole bereits beschrieben worden (vgl. Chem. Abstr. 63, 8344 c (1965) und Chem. Abstr. 85, 62 996 t). Biologische Eigenschaften dieser Stoffe sind aber bisher nicht bekannt.

Es wurde nun gefunden, daß die teilweise bekannten Phenylsulfonylazole der Formel 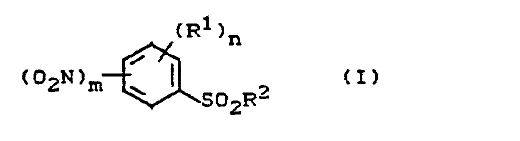 in welcher
R¹ für Alkyl steht,
m für die Zahlen 1 oder 2 steht,
n für die Zahlen 0 oder 1 steht und
R² für einen Rest der Formel 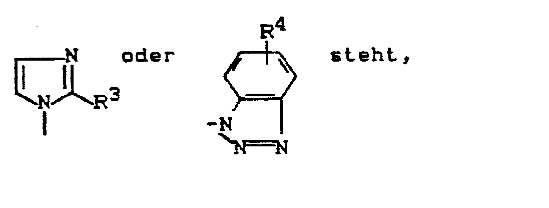 worin
R³ für Alkyl steht und
R⁴ für Wasserstoff, Halogen, Nitro oder Alkyl steht,

sehr gut als Mikrobizide verwendbar sind.

Überraschenderweise besitzen die erfindungsgemäß verwendbaren Phenylsulfonylazole der Formel (I) eine bessere fungizide Wirksamkeit als das N-Trichlormethylthiotetrahydrophthalimid, welches ein konstitutionell ähnlicher, vorbekannter Wirkstoff gleicher Wirkungsart ist. Außerdem übertreffen die Phenylsulfonylazole der Formel (I) auch das bekannte 1-(4-Methyl-phenylsulfonyl)-imidazol bezüglich der fungiziden Eigenschaften.

Die erfindungsgemäß verwendbaren Phenylsulfonylazole sind durch die Formel (1) allgemein definiert. In dieser Formel stehen vorzugsweise
R¹ für Alkyl mit 1 bis 6 Kohlenstoffatomen,
m für die Zahlen 1 und 2,
n für die Zahlen 0 und 1 und
_{R}² für einen Rest der Formel 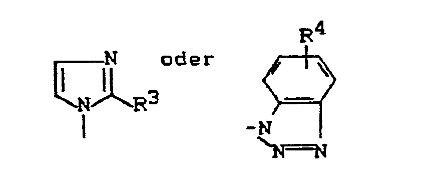 worin
R³ für Alkyl mit 1 bis 4 Kohlenstoffatomen steht und
R⁴ für Wasserstoff, Fluor, Chlor, Brom, Nitro oder für Alkyl mit 1 bis 4 Kohlenstoffatomen steht.

Besonders bevorzugt verwendbar sind diejenigen Verbindungen der Formel (I),
in welcher
R¹ für Alkyl mit 1 bis 4 Kohlenstoffatomen steht,
m für die Zahlen 1 und 2 steht,
n für die Zahlen 0 und 1 steht und
R² für einen Rest der Formel 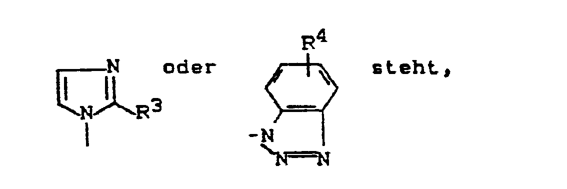 worin
R³ für Methyl oder Ethyl steht und,
R⁴ für Wasserstoff, Fluor, Chlor, Brom, Methyl, Ethyl, n-Propyl, i-Propyl oder Nitro steht.

Ganz besonders bevorzugt verwendbar sind die Verbindungen der Formel (I),
in welcher
R¹ für Methyl, Ethyl oder n-Propyl steht,
m für die Zahlen 1 und 2 steht,
n für die Zahlen 0 und 1 steht und
R² für einen Rest der Formel 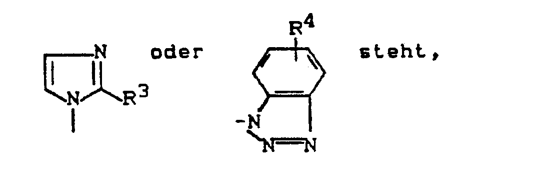 worin
^{R3} für Methyl steht und
R⁴ für Wasserstoff, Chlor, Nitro oder Methyl steht. Von besonderer Bedeutung sind die Verbindugnen der Formel 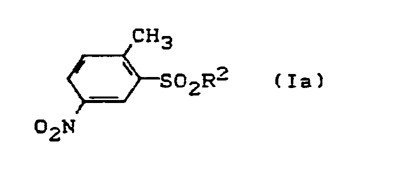 in welcher
R² für einen Rest der Formel 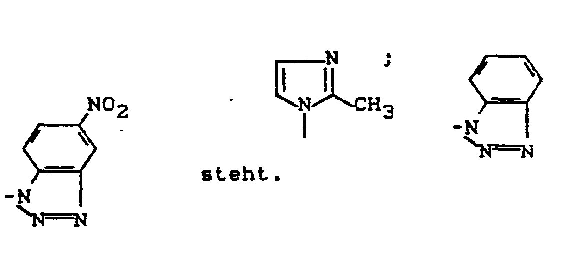 oder

Die erfindungsgemäß verwendbaren Phenylsulfonylazole der Formel (I) sind teilweise bekannt (vgl. Chem. Abstr. 63, 8344 c (1965) und Chem. Abstr. 85, 62 996 t).

Die Phenylsulfonylazole der Formel 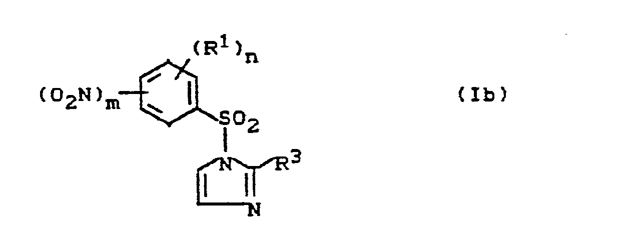 in welcher
_{R}¹ für Alkyl steht,
m für die Zahlen 1 oder 2 steht,
n für die Zahlen 0 oder 1 steht und
_{R}³ für Alkyl steht,

sind neu.

Die Phenylsulfonylazole der Formel (Ib) lassen sich herstellen, indem man Sulfonsäurehalogenide der Formel 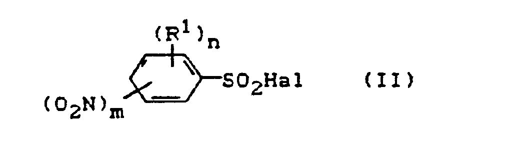 in welcher
R¹, m und n die oben angegebenen Bedeutungen haben und
Hal für Halogen steht,
mit Imidazol-Derivaten der Formel 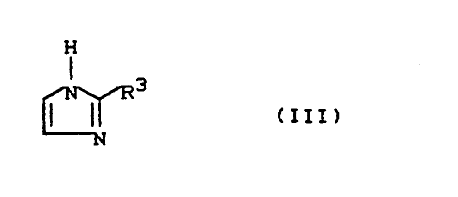 in welcher
R³ die oben angegebene Bedeutung hat,

gegebenenfalls in Gegenwart eines Säureakzeptors und gegebenenfalls in Gegenwart eines Verdünnungsmittels umsetzt.

Die neuen Phenylsulfonylazole sind durch die Formel (Ib) allgemein definiert. In dieser Formel haben R¹, R³, m und n vorzugsweise diejenigen Bedeutungen, die bereits im Zusammenhang mit der Beschreibung der erfindungsgemäß verwendbaren Phenylsulfonylazole der Formel (I) vorzugsweise für diese Reste bzw. diese Indices genannt wurden.

Verwendet man beispielsweise 4-Methyl-3-nitro-benzolsul- fonsäurechlorid und 2-Methylimidazol als Ausgangsstoffe, so kann der Reaktionsablauf des oben beschriebenen Verfahrens durch das folgende Formelschema wiedergegeben werden:

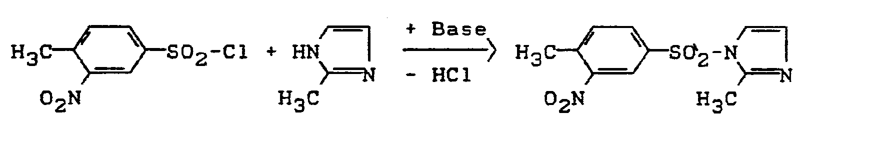

Die bei der Durchführung des oben beschriebenen Verfahrens als Ausgangsstoffe zu verwendenden Sulfonsäurehalogenide sind durch die Formel (II) allgemein definiert. In dieser Formel haben R¹, m und n vorzugsweise diejenigen Bedeutungen, die bereits im Zusammenhang mit der Beschreibung der erfindungsgemäß verwendbaren Stoffe der Formel (I) vorzugsweise für den Rest R^{l} und die Indices m und n genannt wurden. Ha1 steht vorzugsweise für Chlor oder Brom.

Als Beispiele für die Verbindungen der Formel (II) seien genannt:
2-Nitro-, 3-Nitro-, 4-Nitro-, 2-Methyl-3-nitro-, 4-Methyl-3-nitro-, 2-Methyl-5-nitro-, 3,5-Dinitro-2-methyl-, 2-Methyl-4-nitro-, 2-Ethyl-5-nitro-, 2-n-Propyl-5-nitro-, 2-iso-Propyl-5-nitro- und 2-n-Butyl-5-nitro-benzolsulfon- säurechlorid bzw. -bromid.

Die Verbindungen der Formel (II) sind bekannte Verbindungen der organischen Chemie oder können nach allgemein bekannten Verfahren hergestellt werden.

Die beim oben beschriebenen Verfahren außerdem als Ausgangsstoffe zu verwendenden Imidazol-Derivate sind durch die Formel (III) allgemein definiert. In dieser Formel hat R³ vorzugsweise diejenigen Bedeutungen, die bereits im Zusammenhang mit der Beschreibung der erfindungsgemäß verwendbaren Stoffe der Formel (I) vorzugsweise für diesen Substituenten genannt wurden.

Als Beispiele für die Verbindungen der Formel (III) seien genannt:
2-Methyl-, 2-Ethyl-, 2-n-Propyl-, 2-i-Propyl-, 2-n-Butyl-, 2-i-Butyl-, 2-sec.-Butyl- und 2-tert.-Butyl-imidazol.

Die Imidazol-Derivate der Formel (III) sind bekannte Verbindungen der organischen Chemie oder können nach allgemein bekannten Verfahren hergestellt werden.

Das Verfahren zur Herstellung der neuen Phenylsulfonylazole der Formel (Ib) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Dimethyl- und Dibutylether, Glykoldimethylether und Diglykoldimethylether, Tetrahydrofuran und Dioxan, Ketone wie Aceton, Methylethyl-, Methyl-isopropyl- und Methyl-isobutyl-keton, Ester wie Essigsäuremethylester und-ethylester, Nitrile wie z. B. Acetonitril und Propionitril und Pyridin.

Als Säureakzeptoren können bei dem oben beschriebenen Verfahren alle üblicherweise für derartige Umsetzungen verwendbaren Säurebindemittel eingesetzt werden. Vorzugsweise in Frage kommen Alkalicarbonate wie Natrium-und Kaliumcarbonat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-12,2,21-octan (DABCO).

Die Reaktionstemperaturen können bei dem oben beschriebenen Verfahren in einem größeren Bereich variiert werden. Im allgemeinen arbeitet man bei Temperaturen zwischen
-30 °C und +100 °C, vorzugsweise bei Temperaturen zwischen
-20 °C und +60 °C.

Das oben beschriebene Verfahren wird im allgemeinen unter Normaldruck durchgeführt. Es ist jedoch auch möglich, unter erhöhtem oder vermindertem Druck zu arbeiten.

Zur Durchführung des oben beschriebenen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt.

Die Reaktionsprodukte werden nach üblichen Methoden isoliert. So geht man im allgemeinen so vor, daß man die Reaktionsgemische in Wasser gießt und die ausgefallenen Produkte filtriert. Es ist jedoch auch möglich, das Reaktionsgemisch mit verdünnter Säure und Wasser zu waschen und die verbleibende organische Phase einzuengen.

Nicht nur die Phenylsulfonylazole der Formel (Ib), sondern auch die übrigen Stoffe der Formel (I) lassen sich nach dem oben beschriebenen Verfahren herstellen.

Die erfindungsgemäß verwendbaren Wirkstoffe weisen eine starke mikrobizide Wirkung auf und können zur Bekämpfung von unerwünschten Mikroorganismen praktisch eingesetzt werden. Die Wirkstoffe sind vor allem für den Gebrauch als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Spbaerotbeca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- undloder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalacyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 X.

Die erfindungsgemäß verwendbaren Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-X, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-X, vorzugsweise von 0,0001 bis 0,02 X, am Wirkungsort erforderlich.

Die Herstellung von Phenylsulfonylazolen der Formel (I) wird durch die folgenden Beispiele veranschaulicht.

### Herstellungsbeispiele

### Beispiel 1

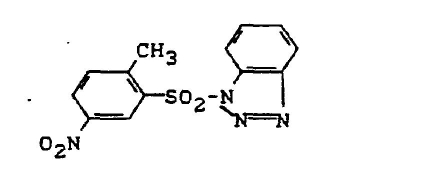

Zu einer Lösung von 5,95 g (0,05 Mol) Benztriazol in 75 ml Pyridin werden bei 20 bis 25°C 11,8 g (0,05 Mol) 2-Methyl-5-nitro-benzolsulfonsäurechlorid zugetropft, und es wird 3 Stunden bei 50°C nachgerührt. Nach dem Abkühlen wird das Reaktionsgemisch auf 500 ml Wasser gegossen und dreimal mit jeweils 50 ml Chloroform extrahiert. Die organischen Phasen werden vereinigt und zweimal mit jeweils 300 ml Wasser gewaschen, anschließend getrocknet und eingeengt. Der RÜckstand wird aus Toluol umkristallisiert.

Man erhält 5,8 g (36,5 % der Theorie) 1-(2-Methyl-5-nitro- phenylsulfonyl)-benztriazol als farblose Kristalle vom Schmelzpunkt 169 bis 170°C.

### Beispiel 2

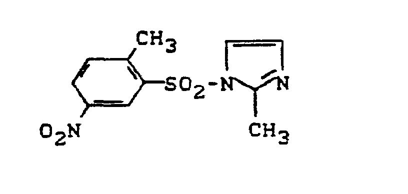

Zu einer Lösung von 82 g (1 Mol) 2-Methylimidazol und 135 g (1,2 Mol) 1,4-Diazabicyclo-[2,2,2]-octan (DABCO) in 2 1 Methylenchlorid werden bei -20°C 235,5 g (1 Mol) 2-Methyl-5-nitro-benzolsulfonsäurechlorid in 500 ml Methylenchlorid zugetropft, und es wird 3 Stunden bei 20 bis 25°C nachgerührt. Anschließend wird das Reaktionsgemisch mit verdünnter Salzsäure und Wasser gewaschen, eingeengt, und der Rückstand wird aus Acetonitril umkristallisiert.

Man erhält 180 g (64 % der Theorie) 2-Methyl-1-(2-methyl-5-nitro-phenylsulfonyl)-imidazol als farblose Kristalle vom Schmelzpunkt 159° C - 161° C.

Analog Beispiel 1 und 2 können die folgenden Verbindungen der allgemeinen Formel 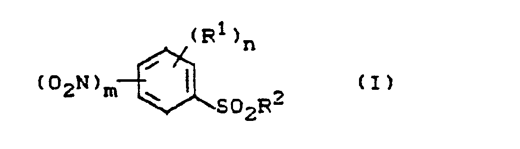 hergestellt werden:

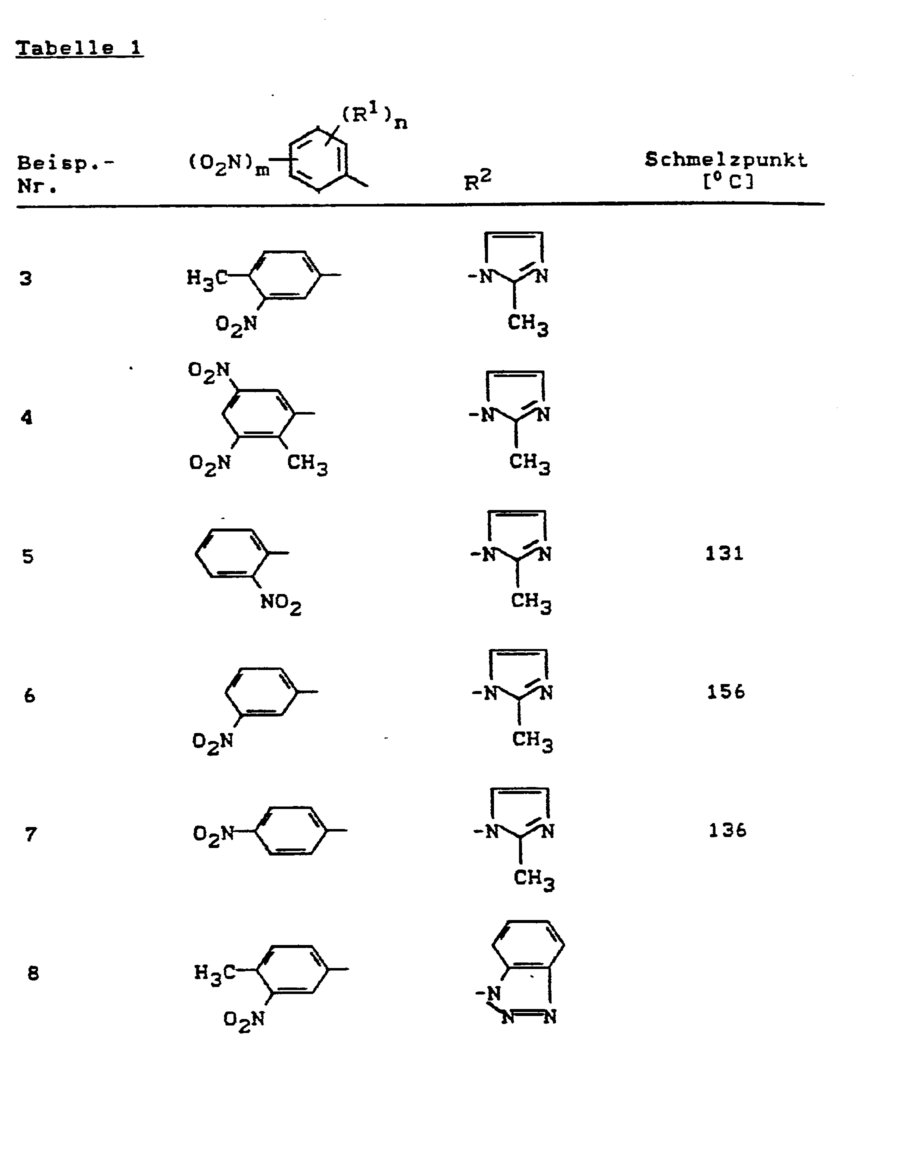

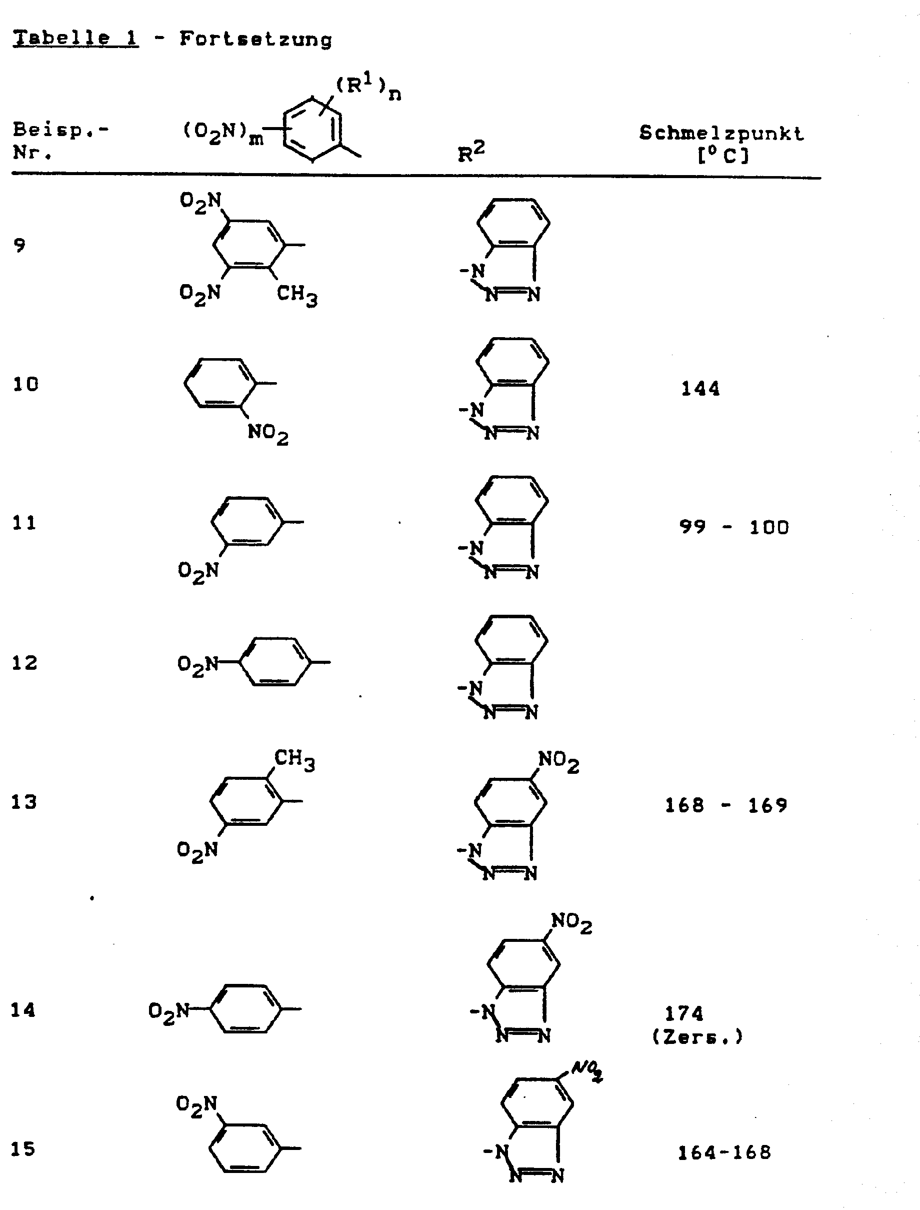

### Beispiel A

### Phytophthora-Test (Tomate) /protektiv

Lösungsmittel: 4,7 Gewichtsteile Aceton
Emulgator: 0,3 Gewichtsteile Alkylaryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

Wirkstoffe, Wirkstoffkonzentrationen und Versuchsergebnisse gehen aus der folgenden Tabelle hervor.

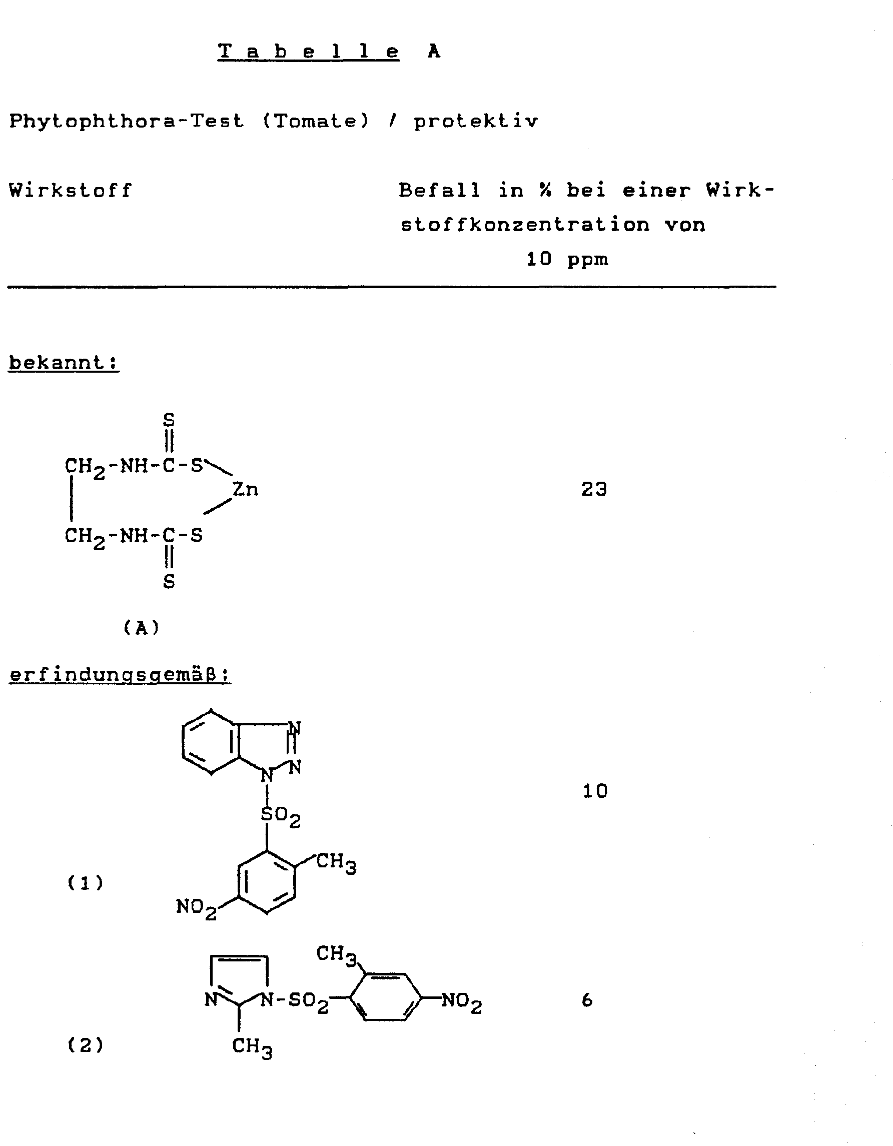

### Beispiel B

### Phytophthora-Test (Tomate) / kurativ

Lösungsmittel: 4,7 Gewichtsteile Aceton
Emulgator: 0,3 Gewichtsteile Alkylaryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20⁰C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einer Inkubationskabine mit 100 % relativer Luftfeuchtigkeit und ca. 20°C aufgestellt.

3 Tage nach der Inokulation erfolgt die Auswertung.

Wirkstoffe, Wirkstoffkonzentrationen und Versuchsergebnisse gehen aus der folgenden Tabelle hervor.

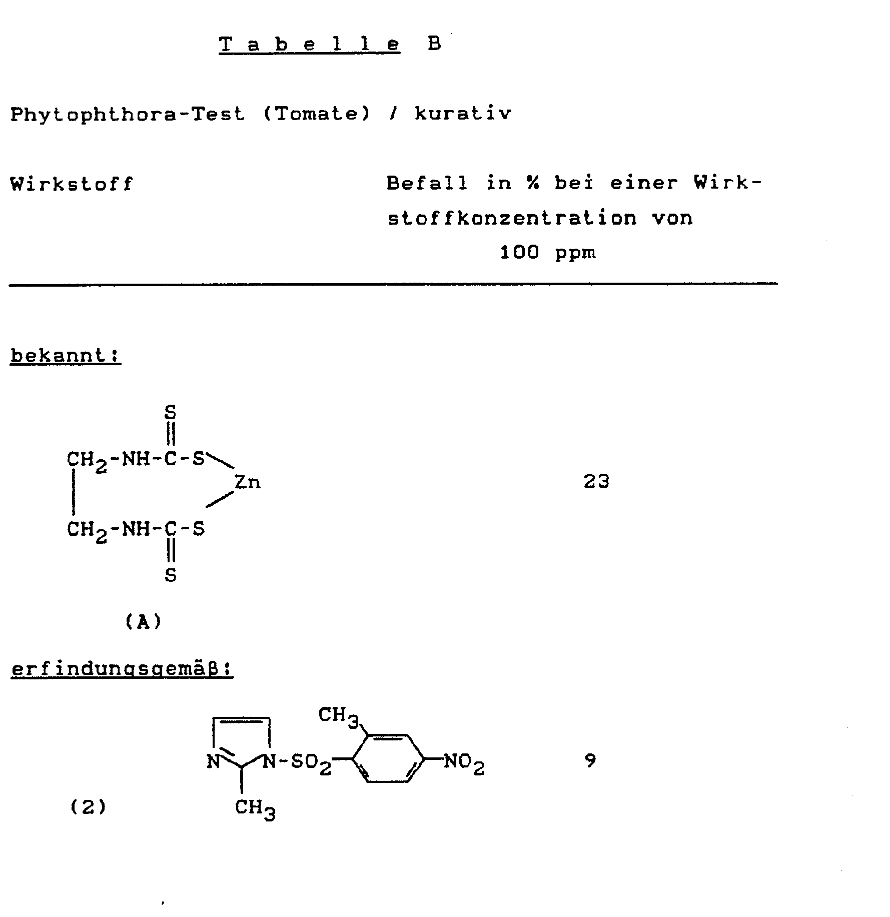

## Patentansprüche

1. Mikrobizide Mittel, gekennzeichnet durch einen Gehalt an mindestens einem Phenylsulfonylazol der Formel 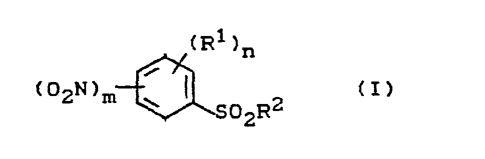
in welcher
R¹ für Alkyl steht,
m für die Zahlen 1 oder 2 steht,
n für die Zahlen 0 oder 1 steht und
R² für einen Rest der Formel 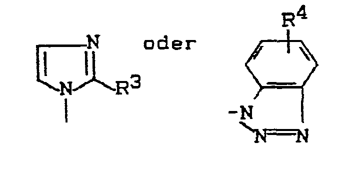 steht,
worin
_{R}³ für Alkyl steht und
R⁴ für Wasserstoff, Halogen, Nitro oder Alkyl steht.

2. Mittel gemäß Anspruch 1, gekennzeichnet durch einen Gehalt an mindestens einem Phenylsulfonylazol der Formel (I),
in welcher
R¹ für Alkyl mit 1 bis 6 Kohlenstoffatomen steht,
m für die Zahlen 1 oder 2 steht,
n für die Zahlen 0 oder 1 steht und
R² für einen Rest der Formel 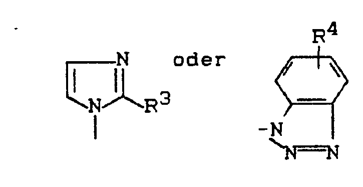 steht,
worin
R³ für Alkyl mit 1 bis 4 Kohlenstoffatomen steht und
R⁴ für Wasserstoff, Fluor, Chlor, Brom, Nitro oder für Alkyl mit 1 bis 4 Kohlenstoffatomen steht.

3. Verfahren zur Bekämpfung von unerwünschten Mikroorganismen, dadurch gekennzeichnet, daß man Phenylsulfonylazole der Formel (I) auf die Mikroorganismen und/oder deren Lebensraum ausbringt.

4. Verwendung von Phenylsulfonylazolen der Formel (I) als Mikrobizide.

5. Verwendung von Phenylsulfonylazolen der Formel (1) als Fungizide.

6. Verfahren zur Herstellung von mikrobiziden Mitteln, dadurch gekennzeichnet, daß man Phenylsulfonylazole der Formel (I) mit Streckmitteln und/oder oberflächenaktiven Stoffen vermischt.

7. Phenylsulfonylazole der Formel 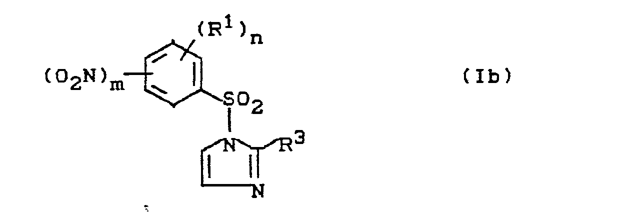
in welcher
_{R}¹ für Alkyl steht,
m für die Zahlen 1 oder 2 steht,
n für die Zahlen 0 oder 1 steht und
_{R}³ für Alkyl steht.

8. Verfahren zur Herstellung von Phenylsulfonylazolen der Formel 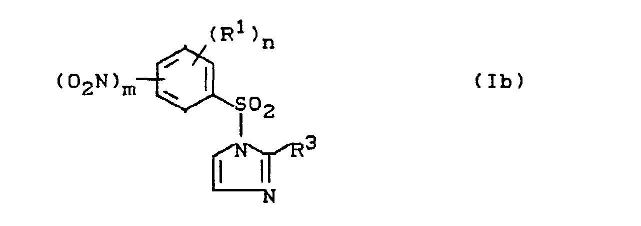
in welcher
_{R}¹ für Alkyl steht,
m für die Zahlen 1 oder 2 steht,
n für die Zahlen 0 oder 1 steht und
_{R}³ für Alkyl steht.
dadurch gekennzeichnet, daß man Sulfonsäurehalogenide der Formel 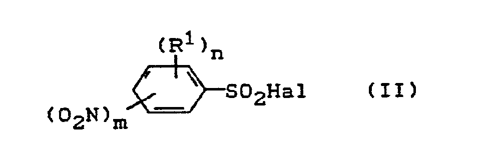
in welcher
R¹, m und n die oben angegebenen Bedeutungen haben und
Hal für Halogen steht,
mit Imidazol-Derivaten der Formel 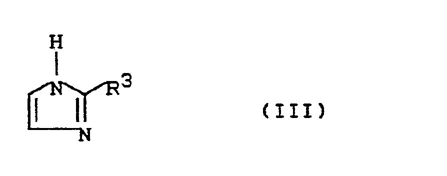
in welcher
R³ die oben angegebene Bedeutung hat,
gegebenenfalls in Gegenwart eines Säureakeptors und gegebenenfalls in Gegenwart eines Verdünnungsmittels umsetzt.
